# EUROPÄISCHE PATENTSCHRIFT

(11) **EP 1 705 172 B1**
(45) Veröffentlichungstag und Bekanntmachung des Hinweises auf die Patenterteilung: **10.01.2007**
(21) Anmeldenummer: 05006515.0
(22) Anmeldetag: 24.03.2005
(51) Int. Cl.: C07C 217/28, C11D 1/00, C11D 1/66, C11D 1/42, C11D 3/00

(54) **Hydroxylgruppen enthaltende Tenside mit geringer Oberflächenspannung und deren Verwendung**
Hydroxylgroups containing detergents with low surface tension and use thereof
Tensioactifs contenant des fonctions d'hydroxyle avec une faible tension de surface

(43) Veröffentlichungstag der Anmeldung: 27.09.2006
(73) Patentinhaber: Goldschmidt GmbH, 45127 Essen (DE)
(72) Erfinder: Hinrichs, Petra, 44866 Bochum (DE); Lehmann, Kathrin, 51377 Leverkusen (DE); Ulrich-Brehm, Isabella, Dr., 40882 Ratingen (DE); Weyershausen, Bernd, Dr., 45127 Essen (DE)

(56) Entgegenhaltungen:
- EP-A- 1 277 829
- EP-A- 1 405 669
- EP-A- 1 505 146
- US-A1- 2003 153 631

## Beschreibung

Gegenstand der Erfindung sind Hydroxylgruppen enthaltende Tenside mit geringer Oberflächenspannung und deren Verwendung als Tensid in wässrigen Beschichtungsformulierungen.

Wasserbasierte Farben und Lacke werden in großem Maßstab industriell verwendet. Maßgeblich für eine gute Benetzung des Substrates ist es, dass die Oberflächenspannung des wässrigen Systems mit Hilfe eines Tensids abgesenkt wird. Dabei ist es nicht nur entscheidend, dass die statische Oberflächenspannung auf einen niedrigen Wert abgesenkt wird, sondern auch die dynamische. Insbesondere ist eine geringe dynamische Oberflächenspannung für Hochgeschwindigkeits-Applikationen erforderlich, z.B. beim Aufsprühen von Beschichtungen oder im Bereich von Druckprozessen. Darüber hinaus dürfen die verwendeten Tenside die Ausbildung eines gleichmäßigen Filmes nicht stören, keine Trübung verursachen und sie sollten schwach schäumend sein, d.h. den Aufbau großer Mengen von Schaum nicht begünstigen.

Unter wasserbasierten Farben und Lacken sind dabei auch solche Formulierungen zu verstehen, die als wässrige Systeme neben Wasser auch Anteile an organischen Lösemitteln enthalten, wie es z.B. durch die Verwendung von Glycolen als Frostschutzmittel oder in Form von Feuchthaltemitteln genutzt in wässrigen Pigmentpasten oder auch als Filmbildehilfsmittel zum Absenken der Mindestfilmbildetemperatur vieler wässriger Dispersionen, zwingend erforderlich ist.

Nichtionische Tenside wie z.B. Alkylaryl- oder Alkohol-Ethoxylate oder Ethylenoxid (EO)-Propylenoxid (PO)-Copolymere sind zwar in der Lage, die statische Oberflächenspannung hervorragend zu reduzieren, jedoch ist es mit diesen Verbindungsklassen, aufgrund ihres hohen Molekulargewichtes und der daraus resultierenden geringen Molekülbeweglichkeit nicht möglich, die dynamische Oberflächenspannung auf einen für den Anwender akzeptablen Wert zu verringern.

Im Gegensatz dazu vermögen einige anionische Tenside wie die Natriumsalze von Mono- oder Dialkylsulfosuccinaten zwar die dynamische Oberflächenspannung gut zu reduzieren, ihre Verwendung führt jedoch zu einem starken Aufbau von Schaum während der Applikation und die fertige Beschichtung reagiert empfindlich auf Wasser.

In jüngerer Zeit kam es zur Entwicklung einer neuen Klasse von Tensiden, die auf acetylenischen Glykolen und ihren Alkoxylaten beruhen. Die Eigenschaften dieser Tenside liegen zwischen denen der zuvor geschilderten Tenside. Mit diesen Tensiden ist es möglich, sowohl die statische als auch die dynamische Oberflächenspannung zu reduzieren, wobei die erreichbaren Werte nicht ganz an die der nichtionischen und anionischen zuvor genannten Gruppen von Tensiden heranreichen. Dafür sind Formulierungen mit diesen Tensiden vergleichsweise schaumarm (EP-B-0 897 744, US-2 997 447).

Aufgrund dieser Eigenschaften haben sich derartige Tenside in zahlreichen Anwendungen etablieren und durchsetzen können. Dabei werden die Eigenschaften hauptsächlich zurückgeführt auf den starren acetylenischen Alkylspacer, der dafür Sorge trägt, dass es durch die eingeschränkten Freiheitsgrade zu einer Art Vororientierung von polaren und unpolaren Gruppen kommt. Darüber hinaus wurde der geringe Abstand der polaren Gruppen und das geringe Molekulargewicht (< 300 g/mol), das eine hohe Beweglichkeit der Tensidmoleküle zulässt, für die Eigenschaften verantwortlich gemacht.

Problematisch an Verbindungen diesen Typs ist, dass es in Anwendungen nach recht kurzer Zeit erneut zu einem Schaumaufbau kommt. Für den Anwender ist es jedoch von großer Bedeutung, diesen Neuaufbau über einen möglichst langen Zeitraum zu verhindern. Andernfalls müssen Entschäumer zudosiert werden, die unter anderem zu unerwünschten Störungen in der Ausbildung des Lackfilms und Problemen bei der Zwischenlagenhaftung führen können.

Darüber hinaus ist die ökotoxikologische Bewertung von Produkten auf Basis von 2,4,6,8-Tetramethyl-5-decindiol nicht unproblematisch und die Produkte sind darüber hinaus zumindest mit "Xi" (reizend) gekennzeichnet. Dem Lackhersteller stehen aus dieser Substanzklasse entweder nur feste Produkte zur Verfügung oder die Substanz wird für eine einfache Handhabung als 50 %ige Lösung in verschiedenen Lösemitteln wie z.B. Ethylenglycol (Einstufung mit "Xn" (gesundheitsschädlich), steht im Verdacht, fruchtschädigend zu sein) angeboten. Alkoxylate dieser Substanzen sind zwar ebenfalls wirksam, zeigen aber ein deutlich geringeres Potenzial hinsichtlich der Schaumverhinderung.

Produkte, die als Tenside in niedrigviskosen wässrigen oder lösemittelhaltigen Lacken, Farben und Beschichtungsstoffen Einsatz finden, sollten bevorzugterweise 100 %ige flüssige Substanzen sein.

Es wird somit deutlich, dass der Bedarf an umweltfreundlichen, ökotoxikologisch positiv bewertbaren Tensiden gerade für wässrige Beschichtungssysteme im Hinblick auf Schaumverhinderung und -inhibierung bisher nicht strukturell gelöst ist. Die Optimierung einzelner Eigenschaften ist zwar möglich, jedoch in der Regel zu Lasten der anderen erforderlichen Parameter.

Es bestand daher der Bedarf, das Gesamteigenschaftsprofil zu verbessern und Verbindungen zur Verfügung zu stellen, die sowohl eine gute Reduktion der statischen und dynamischen Oberflächenspannung ermöglichen, als auch den Aufbau/Neuaufbau von Schaum über einen langen Zeitraum wirkungsvoll verhindern.

In dem Bestreben, die Nachteile des Standes der Technik zu überwinden und Verbindungen bereitzustellen, die die statische wie die dynamische Oberflächenspannung deutlich reduzieren und gleichzeitig die Bildung/Neubildung von Schaum über einen langen Zeitraum effektiv inhibieren, wurde nun überraschenderweise gefunden, dass dieses Ziel durch spezielle Hydroxylgruppen enthaltende Tenside, herstellbar durch Umsetzung von Alkoholen mit Glycidylverbindungen bzw. durch Umsetzung von mindestens einen Aminwasserstoff enthaltenden Aminen mit Glycidylverbindungen erreicht werden kann.

Ein Gegenstand der Erfindung sind Etheralkohole der allgemeinen Formeln (IV) und (V) 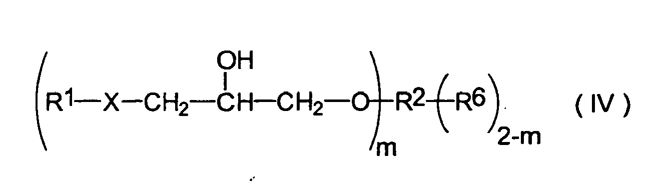 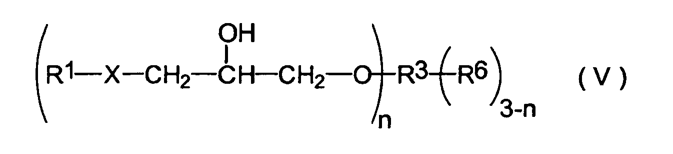 worin
- X: einer Carboxylgruppe entspricht,
- R¹: ein gegebenenfalls verzweigter, gegebenenfalls Heteroatomsubstituenten enthaltender, gegebenenfalls aromatischer, gegebenenfalls ungesättigter Rest mit 1 bis 30, bevorzugt 2 bis 20, besonders bevorzugt 3 bis 9 C-Atomen ist,
- R² und R³: unabhängig voneinander einen gegebenenfalls verzweigten, gegebenenfalls Heteroatomsubstituenten enthaltenden, gegebenenfalls eine oder mehrere Doppel- und/oder Dreifachbindungen enthaltenden, gegebenenfalls (Poly)Oxyalkyleneinheiten, vorzugsweise Oxyethylen- und Oxypropyleneinheiten enthaltenden, gegebenenfalls aromatischen Rest mit 1 bis 30, bevorzugt 2 bis 20 C-Atomen, besonders bevorzugt 3 bis 16 C-Atomen darstellt
- R⁶: beliebige Reste aus der Gruppe der gegebenenfalls verzweigten, gegebenenfalls Heteroatomsubstituenten tragenden, gegebenenfalls ungesättigten Reste sind,
- m: 1 bis 2, vorzugsweise > 1, 5 bis 2 und insbesondere ca. 2,
- n: 2 bis 3, vorzugsweise > 2,5 bis 3 und insbesondere ca. 3 bedeuten.

Ein weiterer Gegenstand der Erfindung sind Aminoalkohole der allgemeinen Formeln (VI) und (VII) 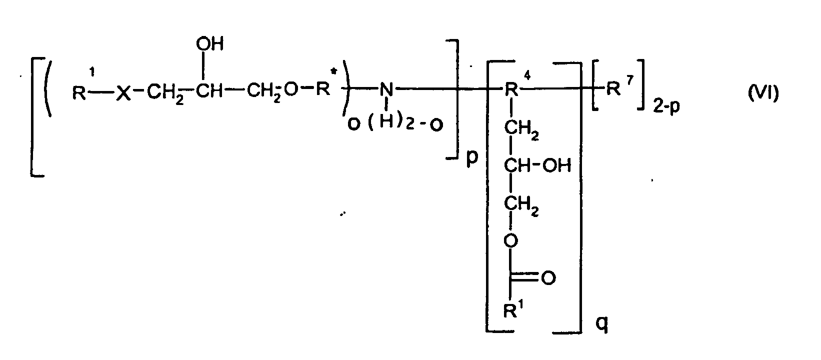 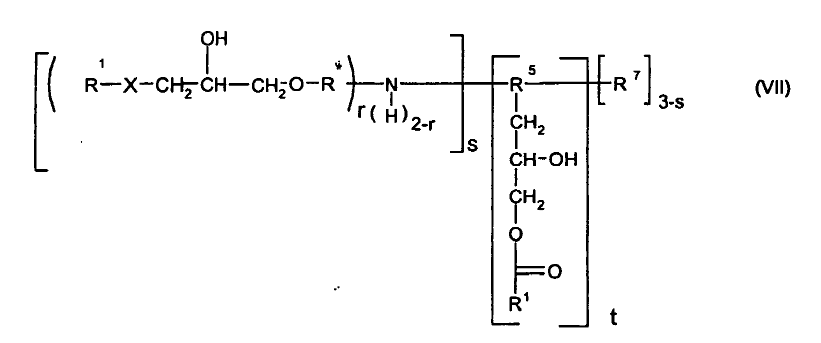 worin
- X: einer Carboxylgruppe entspricht,
- R*: der Rest des 6-Amino-1-hexanol(-(CH₂)₆-), Ethanolamins (-C₂H₄-) oder Isopropanolamins (i-C₃H₆-) ist,
- R¹: ein gegebenenfalls verzweigter, gegebenenfalls Heteroatomsubstituenten enthaltender, gegebenenfalls aromatischer, gegebenenfalls ungesättigter Rest mit 1 bis 30, bevorzugt 2 bis 20, besonders bevorzugt 3 bis 9 C-Atomen ist,
- R⁴: und R⁵ unabhängig voneinander einen gegebenenfalls verzweigten, gegebenenfalls Heteroatomsubstituenten enthaltenden, gegebenenfalls ungesättigten, gegebenenfalls (Poly)Oxyalkyleneinheiten, vorzugsweise Oxyethylen- und Oxypropyleneinheiten enthaltenden, gegebenenfalls aromatischen Rest mit 1 bis 30, bevorzugt 2 bis 20 C-Atomen, besonders bevorzugt 3 bis 8 C-Atomen darstellt,
- R⁷: beliebige Reste aus der Gruppe der gegebenenfalls verzweigten, gegebenenfalls Heteroatomsubstituenten tragenden, gegebenenfalls ungesättigten Reste sind,
- o: 1 oder 2,
- p: 1 bis 2, vorzugsweise > 1,5 bis 2 und insbesondere ca. 2 bedeuten,
- q: entweder 0 ist oder der Anzahl an -NH-Gruppen in R⁴ aus Formel (VI) entspricht,
- r: 1 oder 2,
- s: 2 bis 3, vorzugsweise > 2,5 bis 3 und insbesondere ca. 3 bedeuten,
- t: entweder 0 ist oder der Anzahl an -NH-Gruppen in R⁵ aus Formel (VII) entspricht.

Ein weiterer Gegenstand der Erfindung ist die Verwendung der erfindungsgemäßen Etheralkohole und/oder Aminoalkohole als Additive in wässrigen Formulierungen für insbesondere Oberflächenbeschichtungen, Anstrichmittel, Druckfarben oder Lacke.

Ein weiterer Gegenstand der Erfindung sind wässrige Formulierungen, enthaltend mindestens einen der erfindungsgemäßen Etheralkohole und/oder Aminoalkohole, wobei üblicherweise derartige Netzmittel in Mengen von 0,05 % bis 5 %, vorzugsweise von 0,1 % bis 3 % eingesetzt werden.

Die erfindungsgemäß verwendeten Alkohole, Amine, Aminoalkohole und Glycidylether/-ester sind technische Produkte, die in Form ihrer jeweiligen handelsüblichen Spezifikationen einsetzbar sind, wobei in speziellen Anwendungsgebieten der erfindungsgemäßen Etheralkohole und/oder Aminoalkohole aber höhere Reinheitsgrade gefordert werden können.

Besonders bevorzugte Reste R¹ im Epoxid sind lineare oder verzweigte aliphatische, cycloaliphatische, aromatische oder aromatisch-aliphatische Reste mit 3 bis 12 C-Atomen.

Als Glycidylester werden bevorzugt Neodecansäureglycidylester, 2-Ethylhexansäureglycidylester und die Glycidylester verschiedener anderer aliphatischer, gegebenenfalls verzweigter Carbonsäuren, wie Isocarb® 10, Isocarb^{®} 12, Isocarb^{®} 16, Isocarb^{®} 20 (zu Carbonsäuren oxidierte Guerbet-Alkohole von Sasol) eingesetzt.

Als Alkohole werden bevorzugt Isononanol sowie dessen Ethoxylierungs-/Propoxylierungsprodukte, Glycerin, 1,2,6-Hexantriol und Polyalkylenglycole, wie Polyethylenglycol, Polypropylenglycol, Polybutylenglycol, sowie andere Polyalkylenglycole eingesetzt, bei denen es sich um Homo- oder Copolymere mit blockweisem oder statistischem Aufbau handeln kann, deren Alkylengruppen gegebenenfalls verzweigt sind oder aromatische Reste tragen und deren mittleres Molekulargewicht bis zu 1.500 g/mol, besonders bevorzugt zwischen 200 und 1.000 g/mol beträgt.

Als Aminoalkohole werden bevorzugt 6-Amino-1-hexanol, Ethanolamin, Diethanolamin, Triethanolamin, Methyldiethanolamin, Ethyldiethanolamin, Dimethylethanolamin, Diethyllethanolamin, Diisopropanolamin und 2-Dibutylaminoethanol eingesetzt.

Zur Darstellung der beanspruchten Etheralkohole bzw. Aminoalkohole werden Glycidylverbindungen und Alkohole bzw. Amine, bezogen auf Epoxidgruppen und reaktive Hydroxy- bzw. Aminwasserstoffatome in vorzugsweise entweder etwa äquivalenten Mengen oder mit einem Überschuss an Hydroxy- bzw. Aminwasserstoffatomen eingesetzt. Als Berechnungsgrundlage dienen die dem Fachmann geläufigen Werte der OH-Zahl, der Aminzahl und des Epoxidwertes.

### Experimenteller Teil:

Der vollständige Umsatz aller Reaktionen wurde durch ¹H-NMR-Messungen verifiziert.

### Beispiel 1:

### Umsetzung von Neodecansäureglycidylester mit Isononanol, verethert mit Ethylen- und Propylenoxid, welche als handelsübliche Produkte unter der jeweiligen Bezeichnung der Hersteller verfügbar sind

59,2 g (0,13 mol) mit EO und PO verethertes Isononanol (OH-Zahl: 640 mg KOH/g Substanz) und 0,2 g (0,2 Gew.-%) BF₃-Essigsäure werden unter Stickstoff und Rühren auf 50 °C erhitzt. Anschließend werden 30 g (0,13 mol) Neodecansäureglycidylester. (Epoxy-Wert: 6,9 %) langsam zugetropft. Nach beendeter Zugabe wird die Mischung bei 90 °C für 4 Stunden gerührt. Nach beendeter Reaktion wird abgekühlt und man erhält als Produkt eine klare, leicht gelbe Flüssigkeit.

### Beispiel 2:

### Umsetzung von Neodecansäureglycidylester mit Ethanolamin

13,2 g (0,22 mol) Ethanolamin werden unter Stickstoff und Rühren auf 50 °C erhitzt. Anschließend werden 50 g (0,22 mol) Neodecansäureglycidylester (Epoxy-Wert: 6,9 %) langsam zugetropft. Nach beendeter Zugabe wird die Mischung bei 90 °C für 4 Stunden gerührt. Nach beendeter Reaktion wird abgekühlt, und man erhält als Produkt eine klare, farblose Flüssigkeit.

### Beispiel 3:

### Umsetzung von Neodecansäureglycidylester mit 1,2,6-Hexantriol

15 g (0,11 mol) 1,2,6-Hexantriol und 0,19 g (0,2 Gew.-%) BF₃-Essigsäure werden unter Stickstoff und Rühren auf 50 °C erhitzt. Anschließend werden 77,5 g (0,33 mol) Neodecansäureglycidylester (Epoxy-Wert: 6,9 %) langsam zugetropft. Nach beendeter Zugabe wird die Mischung bei 90 °C für 3 Stunden gerührt. Nach beendeter Reaktion wird abgekühlt und man erhält als Produkt eine klare, leicht gelbe Flüssigkeit.

### Beispiel 4:

### Umsetzung von Neodecansäureglycidylester mit Dimethylaminoethanol

19,2 g (0,22 mol) Dimethylaminoethanol werden unter Stickstoff und Rühren auf 50 °C erhitzt. Anschließend werden 0,14 g (0,2 Ges.-%) BF₃-Essigsäure zugegeben und 50 g (0,22 mol) Neodecansäureglycidylether langsam zugetropft. Nach beendeter Zugabe wird die Mischung bei 90 °C für 4,5 Stunden gerührt. Nach beendeter Reaktion wird abgekühlt und man erhält als Produkt eine klare, farblose Flüssigkeit.

### Anwendungstests:

Für die Abprüfung neuer Netzmittel führt ein Fachmann eine Reihe von Tests zur Übersicht durch, um die schauminhibierende bzw. -verhindernde Wirkung ebenso zu beurteilen, wie auch die durch das Tensid initiierte schnelle Schaumzerstörung, wenn sich Schaum durch andere oberflächenaktive Substanzen in einem System gebildet hat. Ein wesentliches weiteres Kriterium zur Einstufung von Netzmittel ist deren Langzeitwirkung im Sinne einer Schaumverhinderung auch nach Lagerung des entsprechenden mit dem Netzmittel ausgestatteten Systems. Diese Fragestellung ist von besonderer Bedeutung, da Schaumverhinderung während der Lackherstellung grundsätzlich von der schaumfreien Applikation mittels Spritzen, Rakeln, Gießen u.a. zu unterscheiden ist, wobei eine Nachdosierung des Tensids während der Applikation unerwünscht ist.

### Dynamische Oberflächenspannung:

Die Bestimmung der dynamischen Oberflächenspannung der formulierten Systeme ist essentiell, um die Geschwindigkeit abschätzen zu können, mit der ein Netzmittelmolekül an eine neuerlich generierte Grenzfläche gelangt, um dadurch aktiv zur Schaumzerstörung beitragen zu können.

Für die Bestimmung dieser Werte wird das online-tensiometer t 60 der Firma SITA Messtechnik GmbH verwandt. Durch das Gerät wird die dynamische Oberflächenspannung nach dem Prinzip des maximalen Blasendrucks gemessen: Durch die innere Anziehungskraft einer Flüssigkeit werden auch Luftblasen, die sich in der Flüssigkeit befinden, komprimiert. Der dabei entstehende Druck steigt mit abnehmendem Blasenradius. Den gegenüber der Umgebung erhöhten Druck macht man sich bei der Blasendruckmethode zunutze. Dazu wird durch eine in eine Flüssigkeit getauchte Kapillare ein Gasstrom geleitet. Die sich dabei bildende Blasenoberfläche wölbt sich und verringert dabei kontinuierlich den Blasenradius. Dabei steigt der Druck bis zu einem Maximaldruck. Bei diesem hat die Blase ihren kleinsten Radius, den Kapillarradius, erreicht und bildet eine Halbkugel. Nach Überschreiten dieses Punktes platzt die Blase auf und reißt von der Kapillare ab, so dass sich eine neue Blase bilden kann. Dabei kommt es zu einem charakteristischen Druckverlauf, der zur Bestimmung der Oberflächenspannung ausgewertet wird. D.h. je kleiner der Wert bei geringer Blasenfrequenz, umso effektiver ist das Netzmittel in der Benetzung einer niedrigenergetischen Oberfläche. Je geringer der Unterschied zwischen dem Wert bei niedriger und hoher Blasenfrequenz, desto besser ist das Netzmittel in der Lage sich an neu geschaffene Oberflächen zu orientieren, d.h. auch während hoch dynamischer Applikationsprozesse wirksam zu sein.
Für die Beurteilung der erfindungsgemäß beanspruchten Netzmittel wurden die im Folgenden näher ausgeführten Tests durchgeführt.

### Schauminhibierende Wirkung:

Zu einer vorgegebenen Menge eines Testsystems wird eine definierte Menge Netzmittel gegeben und mittels Zahnradscheibe 1 Minute bei 1.500 Upm eingearbeitet. Anschließend wird 1 Minute bei 3.000 Upm Luft eingetragen und Schaum erzeugt. Die resultierende Schaumhöhe wird abgelesen und im Vergleich zur Schaumhöhe ohne Verwendung des Netzmittels betrachtet. Im Anschluss daran wird die Zeit gemessen, bis ein vollständiger Abbau des Schaums stattgefunden hat, der ohne Verwendung von Netzmitteln im Allgemeinen gar nicht stattfindet.

### Beurteilung des Schaumaufbaus und der Spontanentschäumung:

In einer vorgegebenen Menge eines Testsystems wird mittels Lochscheibe (siehe unten) 1 Minute bei 2.000 Upm Schaum aufgebaut. Anschließend wird eine definierte Menge Netzmittel auf den Schaum gegeben und das Auftreten von Spontanentschäumung dabei visuell beurteilt (zerplatzende Luftblasen, "Prickeln" auf der Oberfläche) und mit nicht vorhanden (-), vorhanden (+/-) oder sehr charakteristisch (+) aufgeführt.

Anschließend erfolgt eine erneute einminütige Scherung mit der Lochscheibe bei 2.000 Upm. Dabei wird die Zeit gestoppt, bis ein erneuter Schaumaufbau erfolgt. Kann ein Netzmittel den erneuten Schaumaufbau verhindern, ist es mit "> 60 s" als sehr aktiv einzustufen.

Eine definierte Menge dieser Probe wird anschließend in einen Messzylinder gegeben und die Schaumhöhe wird durch die Angabe der ml Schaum festgehalten und vergleichend zur Blindprobe betrachtet.

Bei der verwandten Lochscheibe handelt es sich um drei an einer Achse übereinander angeordnete Scheiben (Dicke 3 mm, Durchmesser 25 mm) mit je drei Löchern (Durchmesser 5 mm). Der Abstand der Einzelscheiben zueinander beträgt 9 mm und sie weisen an der Befestigungsachse eine vertikale Drehung um 120 ° auf. Dieses Aggregat ermöglicht einen optimalen Eintrag von Makro- und Mikroschaum, wie er bei Lackierapplikationsprozessen (wie z.B. Walzen, Spritzen) und Herstellprozessen auftritt und durch geeignete Netzmittel zu verhindern ist.

### Langzeitwirkung:

Nach einer Lagerung der zweifach scherbelasteten Probe (siehe oben beschriebener Test) von 4 bis 14 Tagen wird die Probe erneut mit der Lochscheibe, 1' bei 2.000 Upm, gerührt und nochmals die sich ergebende Schaumhöhe einer Probe in einem Messzylinder abgelesen. Weichen diese Werte kaum von der Erstbestimmung ab, steht das Netzmittel weiterhin im System zur Verfügung und zeichnet sich also auch als hydrolysestabil aus.

### Viskosität:

Häufig treten durch in Farben, Lacke und Beschichtungsstoffe eingearbeitete Tenside unerwünschte Änderungen in der Viskosität des Systems auf, was dann durch Eindickeffekte beispielsweise zu grundsätzlich anderen Schichtdicken während der Applikation führt und somit die Wirtschaftlichkeit in Frage gestellt wird. Es muß daher das mit dem Tensid additivierte Beschichtungsstoffsystem im Vergleich zu der nicht scherbelasteten Blindprobe ohne Additiv bewertet werden. Dazu stehen verschiedene Rheometer zur Verfügung, wobei hier das RC 20-CPS von Europhysics genutzt wird. Das angewandte Programm misst von 100 [l/s] auf 1.000 [l/s] in 180 Sekunden mit einer Platte-Kegel-Geometrie.

### Unverträglichkeit:

In transparenten Systemen ist bereits eine geringfügige Trübung ein Hinweis auf eine Unverträglichkeit des Tensids in der umgebenden Matrix, welche unerwünscht ist. Um zu sichern, dass die schaumverhindernde Wirkung des Tensids nicht auf Kosten von getrübten Klarlacken oder durch Kraterbildung bezahlt wird, appliziert der Fachmann den entsprechenden Lack auf verschiedenen Substraten zur visuellen Bewertung (z.B. schwarze PVC-Folie oder transparente PE-Folie).

In den folgenden Tests werden die erfindungsgemäßen Netzmittel mit S1 bis S7 bezeichnet.
S4 Beispiel 9
S5 Beispiel 1
S6 Beispiel 2

Nichterfindungsgemäße Vergleichsbeispiele sind die nachfolgenden Netzmittel, die als handelsübliche Produkte für wässrige Systeme angeboten werden und entsprechend den unten stehenden Angaben charakterisierbar sind.
V1 2,4,7,9-Tetramethyl-5-decin-4,7-diol, 100 %ig festes Tensid
V2 2,4,7,9-Tetramethyl-5-decin-4,7-diolethoxylat
V3 Fettalkoholalkoxylat mit einem Molgewicht von etwa 500 g/mol
V4 Trisiloxanpolyethertensid
V5 Oligosiloxanpolyethertensid
V6 styroloxidbasiertes Blockcopolymer mit dem Molgewicht von etwa 1.000 g/mol.

Die vorgenannten erfindungsgemäßen und handelsüblichen Netzmittel werden zur Verwendung in den folgenden üblichen Formulierungen herangezogen und die Reduzierung der Oberflächenspannung in wässriger 0,5 %igen Tensidlösungen bestimmt.

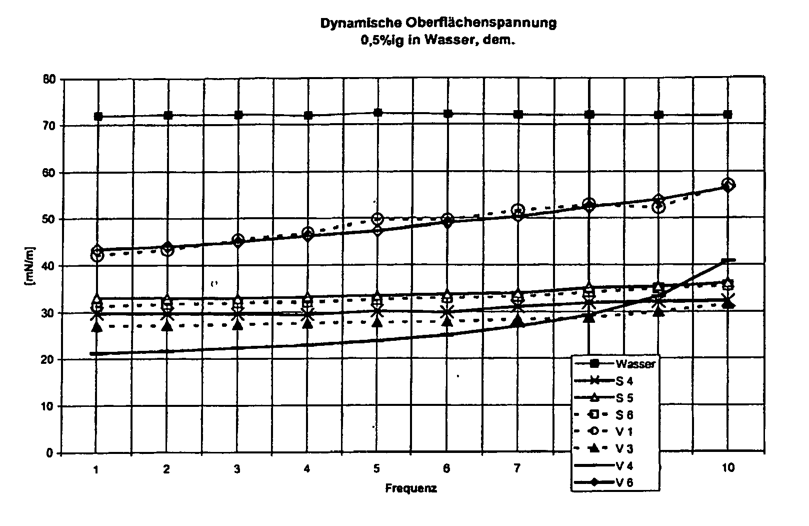

### Wasserbasierte Druckfarbenformulierung:

In 50 g Farbe bestehend aus:

| | |
|---|---|
| JonCryl^{®} 8085 (43 %ige | |
| ammoniakalische Lösung | |
| eines Acrylatharzes)¹⁾ | 29,4 g |
| JonCryl^{®} ECO 2189 (glycol | |
| etherfreie filmbildende | |
| Polymerdispersion)¹⁾ | 44,1 g |
| JonCryl^{®} ECO 2177 (glycol | |
| etherfreie filmbildende | |
| Polymerdispersion)¹⁾ | 17,7 g |
| JonWax^{®} 35 (Poly | |
| ethylenwachsemulsion)¹⁾ | 4,9 g |
| demineralisiertes Wasser | 2,9 g |

| | |
|---|---|
| ¹⁾ Johnson Polymer | |

werden in einer 100 ml Glasflasche eingewogen und mit einer 2,5 cm-Zahnrandscheibe 1' bei 1.500 Upm 0,5 % Wirkstoff Netzmittel eingerührt und anschließend 1' bei 3.000 Upm aufgeschäumt. Die Füllhöhe (Lösung + Schaum) wird mittels Lineal in der Glasflasche abgelesen und die Zeit des Schaumzusammenbruchs in Minuten mittels Stoppuhr bestimmt.

**Tabelle 1:**

| Ergebnisse in einer wasserbasierten Druckfarbe mit vorangegangener Messung der dynamischen Oberflächenspannung in Wasser: | | |
|---|---|---|
| Netzmittel | Schaum [cm] | Dynamische Oberflächenspannung der 0,5 %igen Tensidlösungen bei 1 und 10 Blasen/sec [mN/m] |
| Ohne | 7,0 | 72 bis 72 |
| S5 | 5,0 | 33,0 bis 36,1 |
| V1 | 6,0 | 42,2 bis 47,0 |
| V2 | 6,0 | - |
| V3 | 6,7 | 30,2 bis 32,6 |
| V4 | - | 21,2 bis 40,8 |
| V6 | - | 43,4 bis 56,5 |

Tabelle 1 zeigt, dass durch die Verwendung der erfindungsgemäß beanspruchten Netzmittel der Schaumaufbau gegenüber der Blindprobe und den Vergleichsbeispielen deutlich reduziert ist. Betrachtet man darüber hinaus die Reduzierung der dynamischen Oberflächenspannung, die die erfindungsgemäß beanspruchten Tenside in Wasser realisieren können, wird deutlich, dass die Kombination der Eigenschaften Schaumreduzierung und Verfügbarkeit an neukreierten Grenzflächen in schnellen Applikationsprozessen wie z.B. bei Druckfarbenapplikationen diese neuartige Tensidklasse auszeichnet.

Sie sind darüber hinaus mit ihrer 100 % reinen flüssigen Darreichungsform einfach einarbeitbar und somit benutzerfreundlich. Die erfindungsgemäß beanspruchten Tenside zeigen also weder eine derartige ungenügende Wasserlöslichkeit und daraus resultierende ungenügende Reduzierung der Oberflächenspannung wie z.B. das Vergleichsbeispiel V1 (fester, hydrophober Stoff) noch sind sie wie beispielsweise V4 bis V6 hinsichtlich der dynamischen Oberflächenspannung limitiert, was besonders bei 10 Blasen/sec relevant für schnelle Applikationsprozesse ist.

### Wasserbasierter Autolack I:

50 g einer Mischung aus 2 Teilen aliphatischer Polyurethan-Acryl-Hybrid-Dispersion Daotan^{®} VTW 6264 (Firma Solutia) und einem Teil VE-Wasser werden eine Minute mit 2.000 Upm in einem Becher (Durchmesser 65 mm) mit einer Lochscheibe (Beschreibung siehe oben) aufgeschäumt. Auf den resultierenden Schaum werden 0,2 % Wirkstoff Netzmittel gegeben und die Spontanentschäumung beobachtet. Anschließend wird erneut 1 Minute bei 2.000 Upm geschert und die Zeit bis zum erneuten Schaumaufbau mittels Stoppuhr ermittelt. Tritt kein erneuter Schaumaufbau auf, ist die Bewertung mit > 60 sec angegeben.

25 g dieser Probe werden im direkten Anschluss an die Scherbelastung in einen 100 ml Messzylinder gegeben und die Füllhöhe in ml abgelesen.

Zur Beurteilung der Hydrolyse- und Lagerstabilität wird die Probe nach vier Tagen erneut 1 Minute bei 2.000 Upm geschert und die Schaumhöhe von 25 g mittels 100 ml Messzylinder ermittelt.

**Tabelle 2:**

| Ergebnisse in einem wässrigen Autolack I: | | | | |
|---|---|---|---|---|
| Netzmittel | Spontanentschäumung* | Wiederaufbau von Schaum [sec] | Schaumwert sofort [ml/25 g] (Restschaum) | Schaumwert nach 4 Tagen [ml/25 g] |
| Blindwert | entfällt | entfällt | 44 | 46 |
| S4 | + | 60 | 30 | 36 |
| S5 | + | 60 | 30 | 31 |
| S6 | + | 60 | 30 | 35 |
| V1 | + | 45 | 30 | 32 |
| V2 | +/- | 60 | 37 | 40 |
| V3 | + | 45 | 30 | 33 |
| V4 | - | - | 50 | 50 |
| V5 | +/- | 10 | 35 | 38 |

| | | | | |
|---|---|---|---|---|
| *(-) nicht vorhanden, (+/-) vorhanden, (+) sehr deutlich ausgeprägt | | | | |

Die erfindungsgemäßen Verbindungen zeigen eine ausgeprägte Spontanentschäumung. Bereits diese Eigenschaft der neuartigen Tenside ist für den Lackhersteller von größtem Interesse. Wählt man dann noch besondere Produkte wie S4 bis S7 aus, ist es darüber hinaus möglich, Tenside zu erhalten, die dann auch noch durch besonders geringe Restschaumwerte auffallen und auch bei weiterem Schereintrag eine lang anhaltende Schaumverhinderung ermöglichen. Damit muss dem Autolacksystem auch nach Lagerung kein zusätzliches Netzmittel oder Entschäumer zugesetzt werden.

Wesentlich in der positiven Bewertung der erfindungsgemäßen Verbindungen ist hier die Summierung der wichtigen Eigenschaften in einer Struktur:
spontane Entschäumung
+ kein oder sehr später Wiederaufbau von Schaum
+ optimale Reduktion des Schaums (sofort + nach Lagerung)

### Wasserbasierter Autolack II:

Auf 10 g eines wasserverdünnbaren, selbstvernetzenden, urethangruppenhaltigem Alkydharz Resydrol VAZ 5541(Firma Solutia) werden 0,5 % Surfactant gewogen und mittels Speedmixer der Firma Hausschild eine Minute bei 3600 Upm eingerührt.

Nach drei Tagen wird die Viskosität der Proben mittels RC 20-CPS Viskosimeter der Firma Europhysics bei 500 Umdrehungen/Sekunde in mPas bestimmt.

Zusätzlich erfolgt ein Aufzug mit 50 µm mittels Kastenrakel auf Aluminium zur Beurteilung der Verträglichkeit.

**Tabelle 3:**

| Ergebnisse in einem wässrigen Autolack II: | | |
|---|---|---|
| Netzmittel | Eindicken [mPas] | Charkterisierung des Lackfilms |
| Blindwert | 124 | +++ |
| S5 | 127 | +++ |
| V1 | 138 | +-- |
| V2 | 132 | --- |
| V3 | 136 | ++- |
| V5 | 129 | +-- |

| | | |
|---|---|---|
| (+++) = ohne Störungen (---) = starke Störungen | | |

Tabelle 3 illustriert mit den entsprechend niedrigen Viskositäten, die für die erfindungsgemäß beanspruchten Verbindungen auf dem Niveau des puren Autolacksystems ohne Netzmittel (Blindwert) liegen, dass so gut wie keine Viskositätserhöhung stattfindet im Gegensatz zu den Vergleichsbeispielen.
Gerade in den sensiblen dünnschichtigen Autolackapplikationen ist damit eine Lösung zum reproduzierbaren Schichtdickenaufbau und Oberflächenbild gefunden. Dabei wird die gute Entlüftung des Systems, die bereits anhand der Viskositäten nachgewiesen werden kann, zusätzlich durch ein sehr gutes Oberflächenbild der Lacke in Form störungsfreier Filme deutlich.

### Fazit:

Die erfindungsgemäß beanspruchten Verbindungen können eindeutig als Netzmittel in wässrigen Lacken, Farben und Beschichtungsstoffen genutzt werden, da sie die Oberflächenspannung signifikant senken, wobei diese wässrigen Lacke und Farben gegebenenfalls Anteile an organischen Lösemitteln enthalten können.
Besonders interessant ist dabei dass sie auch in schnellen Applikationsprozessen als wirksames Netzmittel erkennbar sein werden, da sie ein dynamisches Oberflächenspannungsprofil zeigen, das nahezu unabhängig von der Frequenz, also der Vielzahl der neukreierten Grenzflächen, ist.
Die erfindungsgemäß beanspruchten Hydroxylgruppen enthaltenden Tenside vereinen daher in einem bisher nicht bekannten Eigenschaftsprofil Spontanentschäumungseigenschaften, die während der Lackherstellung und -applikation gebraucht werden, mit starker Schaumzerstörung (keine oder sehr geringe Restschaumwerte). Die zuletzt genannte Eigenschaft ist dabei auch nach Lagerung des Lackes zu finden, was bedeutet, dass die erfindungsgemäß beanspruchten Verbindungen nicht hydrolytisch angegriffen werden, dass heißt überraschend stabil sind. Die erfindungsgemäß beanspruchten Verbindungen realisieren Schaumzerstörung dabei nicht auf Kosten eines gestörten Oberflächenbildes, weshalb selbst sensible Autolackoberflächen störungsfrei appliziert werden können.

## Patentansprüche

1. Etheralkohole der allgemeinen Formeln (IV) und (V) 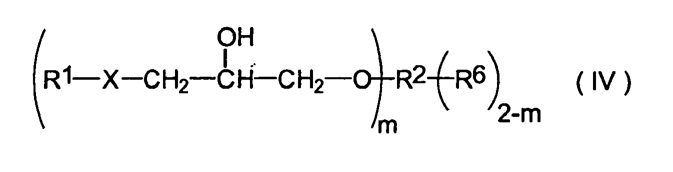 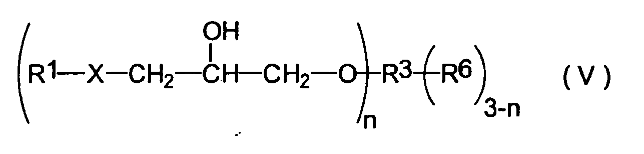 worin
X einer Carboxylgruppe entspricht,
R¹ ein gegebenenfalls verzweigter, gegebenenfalls Heteroatomsubstituenten enthaltender, gegebenenfalls aromatischer, gegebenenfalls ungesättigter Rest mit 1 bis 30 C-Atomen ist,
R² und R³ unabhängig voneinander einen gegebenenfalls verzweigten, gegebenenfalls Heteroatomsubstituenten enthaltenden, gegebenenfalls eine oder mehrere Doppelbindungen enthaltenden, gegebenenfalls (Poly)Oxyalkyleneinheiten, vorzugsweise Oxyethylen- und Oxypropyleneinheiten enthaltenden, gegebenenfalls aromatischen Rest mit 1 bis 30 C-Atomen darstellt,
R⁶ beliebige Reste aus der Gruppe der gegebenenfalls verzweigten, gegebenenfalls Heteroatomsubstituenten tragenden, gegebenenfalls ungesättigten Reste sind,
m 1 bis 2,
n 2 bis 3 bedeuten.

2. Aminoalkohole der allgemeinen Formeln (VI) und (VII) 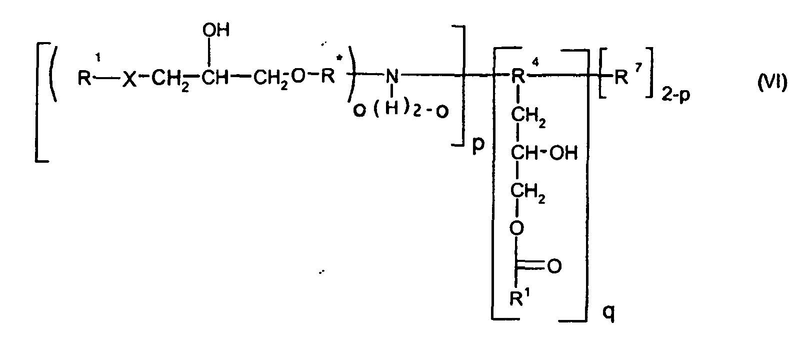 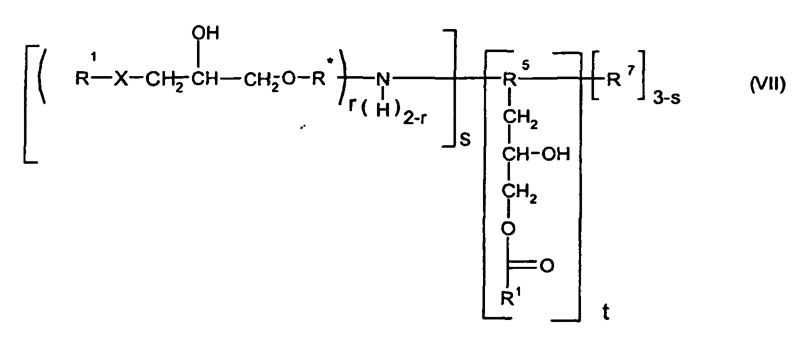 worin
X einer Carboxylgruppe entspricht,
R* der Rest des 6-Amino-1-hexanols(-(CH₂)₆-), Ethanolamins (-C₂H₄-) oder Isopropanolamins (i-C₃H₆-) ist,
R¹ ein gegebenenfalls verzweigter, gegebenenfalls Heteroatomsubstituenten enthaltender, gegebenenfalls aromatischer, gegebenenfalls ungesättigter Rest mit 1 bis 30 C-Atomen ist,
R⁴ und R⁵ unabhängig voneinander einen gegebenenfalls verzweigten, gegebenenfalls Heteroatomsubstituenten enthaltenden, gegebenenfalls ungesättigten, gegebenenfalls (Poly)Oxyalkyleneinheiten enthaltenden, gegebenenfalls aromatischen Rest mit 1 bis 30 C-Atomen darstellt,
R⁷ beliebige Reste aus der Gruppe der gegebenenfalls verzweigten, gegebenenfalls Heteroatomsubstituenten tragenden, gegebenenfalls ungesättigten Reste sind,
o 1 oder 2,
p 1 bis 2 bedeuten,
q entweder 0 ist oder der Anzahl an -NH-Gruppen in R⁴ aus Formel (VI) entspricht,
r 1 oder 2,
s 2 bis 3 bedeuten,
t entweder 0 ist oder der Anzahl an -NH-Gruppen in R⁵ aus Formel (VII) entspricht.

3. Verwendung der erfindungsgemäßen Verbindungen gemäß mindestens einem der Ansprüche 1 oder 2 als Additive in wässrigen Formulierungen für insbesondere Oberflächenbeschichtungen, Anstrichmittel, Druckfarben oder Lacke.

4. Wässrige Formulierungen, enthaltend mindestens eine der erfindungsgemäßen Verbindungen, gemäß mindestens einem der Ansprüche 1 oder 2 in Mengen von 0,05 Gew.-% bis 5 Gew.-%.

## Claims

1. Ether alcohols of the general formulae (IV) and (V) 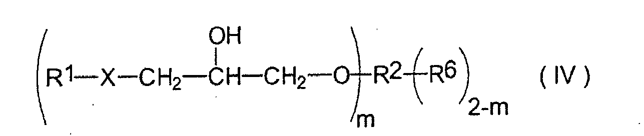 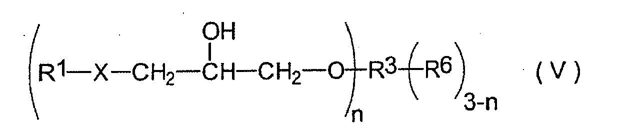 in which
X is a carboxyl group,
R¹ is an unbranched or branched, aromatic or nonaromatic, unsaturated or saturated residue with or without heteroatom substituents and containing 1 to 30 carbon atoms,
R² and R³ independently of one another are an unbranched or branched, aromatic or nonaromatic residue with or without heteroatom substituents, with or without one or more double bonds, with or without (poly)oxyalkylene units, preferably oxyethylene and oxypropylene units, and containing 1 to 30 carbon atoms,
R⁶ are any residues from the group of unbranched or branched, unsaturated or saturated residues with or without heteroatom substituents,
m is 1 to 2 and
n is 2 to 3.

2. Amino alcohols of the general formulae (VI) and (VII) 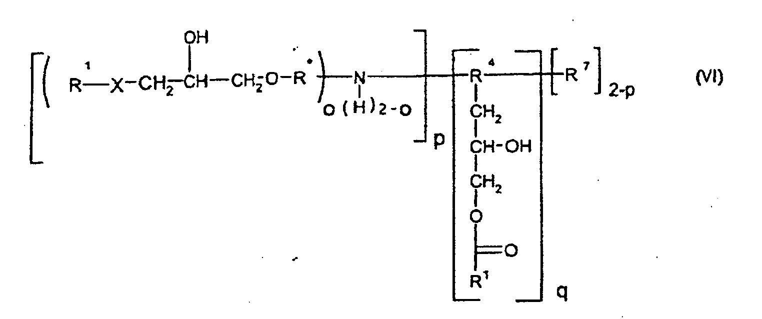 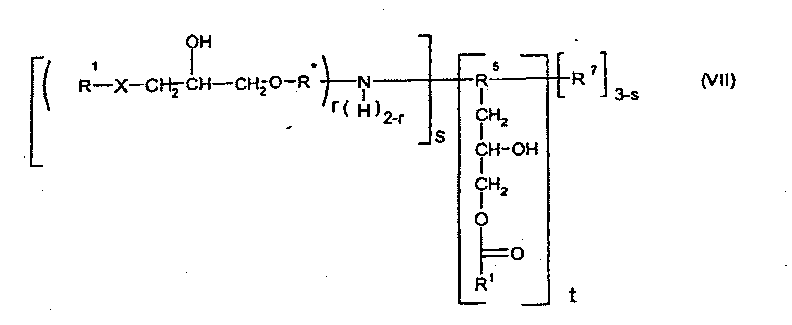 in which
X is a carboxyl group,
R* is the radical of 6-amino-1-hexanol (-CCH₂)₆-) ethanol amine (-C₂H₄-) or isopropanol amine (i-C₃H₆-),
R¹ is an unbranched or branched, aromatic or nonaromatic, unsaturated or saturated residue with or without heteroatom substituents and containing 1 to 30 carbon atoms,
R⁴ and R⁵ independently of one another are an unbranched or branched, unsaturated or saturated, aromatic or nonaromatic residue with or without heteroatom substituents, with or without (poly)oxyalkylene units, and containing 1 to 30 carbon atoms,
R⁷ are any residues from the group of unbranched or branched, unsaturated or saturated residues with or without heteroatom substituents,
o is 1 or 2,
p is 1 to 2,
q is either 0 or corresponds to the number of -NH-groups in R⁴ from formula (VI),
r is 1 or 2,
s is 2 to 3,
t is either 0 or corresponds to the number of -NH-groups in R⁵ from formula (VII).

3. Use of the compounds according to at least one of Claims 1 or 2 as additives in aqueous formulations for, in particular, surface coatings, paints, printing inks or varnishes.

4. Aqueous formulations comprising at least one compound according to at least one of Claims 1 or 2 in amounts from 0.05% to 5% by weight.

## Revendications

1. Etheralcools de formules générales (IV) et (V) 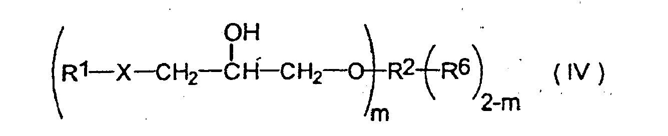 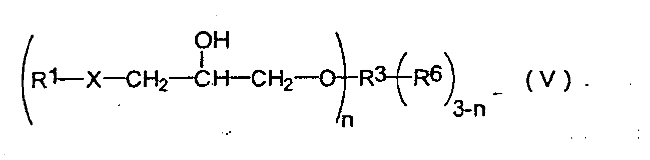 dans lesquelles
X correspond à un groupe carboxyle,
R¹ représente un radical le cas échéant ramifié, contenant le cas échéant des substituants avec des hétéroatomes, le cas échéant aromatique, le cas échéant insaturé, comprenant 1 à 30 atomes de carbone,
R² et R³ représentent, indépendamment l'un de l'autre, un radical le cas échéant ramifié, contenant le cas échéant des substituants avec des hétéroatomes, contenant le cas échéant une ou plusieurs doubles liaisons, contenant le cas échéant des unités (poly)oxyalkylène, de préférence des unités oxyéthylène et oxypropylène, le cas échéant aromatique, comprenant 1 à 30 atomes de carbone,
R⁶ représente des radicaux quelconques du groupe des radicaux le cas échéant ramifiés, portant le cas échéant des substituants avec des hétéroatomes, le cas échéant insaturés,
m vaut 1 à 2,
n vaut 2 à 3.

2. Aminoalcools des formules générales (VI) et (VII) 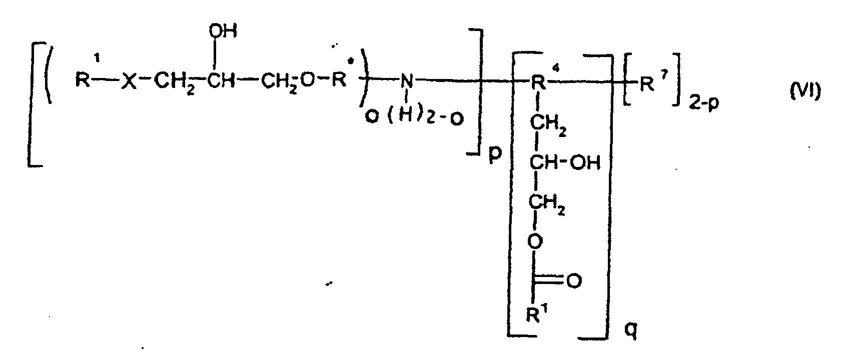 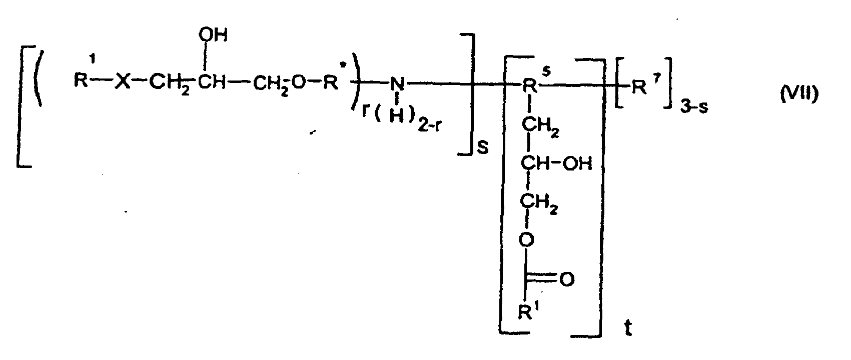 dans lesquelles
X correspond à un groupement carboxyle,
R* est le radical du 6-amino-1-hexanol (-(CH₂)₆-), de l'éthanolamine (-C₂H₄-) ou de l'isopropanolamine (i-C₃H₆-),
R¹ représente un radical le cas échéant ramifié, contenant le cas échéant des substituants avec des hétéroatomes, le cas échéant aromatique, le cas échéant insaturé, comprenant 1 à 30 atomes de carbone,
R⁴ et R⁵ représentent, indépendamment l'un de l'autre un radical le cas échéant ramifié, contenant le cas échéant des substituants avec des hétéroatomes, le cas échéant insaturé, contenant le cas échéant des unités (poly)oxyalkylène, le cas échéant aromatique, comprenant 1 à 30 atomes de carbone,
R⁷ représente des radicaux quelconques du groupe des radicaux le cas échéant ramifiés, portant le cas échéant des substituants avec des hétéroatomes, le cas échéant insaturés,
o vaut 1 ou 2,
p vaut 1 à 2,
q vaut 0 ou le nombre de groupes -NH dans R⁴ de la formule (VI),
r vaut 1 ou 2,
s vaut 2 à 3,
t vaut 0 ou le nombre de groupes -NH dans R⁵ de la formule (VII).

3. Utilisation des composés selon l'invention selon au moins l'une quelconque des revendications 1 ou 2 comme additifs dans les formulations aqueuses pour, en particulier, des revêtements de surface, des enduits, des encres d'imprimerie ou des laques.

4. Formulations aqueuses, contenant au moins un des composés selon l'invention, selon au moins l'une quelconque des revendications 1 ou 2 en des quantités de 0,05% en poids à 5% en poids.
